# EUROPEAN PATENT APPLICATION

(11) **EP 4 704 103 A1**
(43) Date of publication of application: **04.03.2026**
(21) Application number: 24196652.2
(22) Date of filing: 27.08.2024
(51) Int. Cl.: G16H 30/40, G16H 40/63, G16H 40/67

(54) **METHOD, DEVICE AND SYSTEM OF PREPARING A MEDICAL IMAGING EXAMINATION OF A PATIENT**

(71) Applicant: Koninklijke Philips N.V., 5656 AG Eindhoven (NL)
(72) Inventor: SOMMER, Karsten, Eindhoven (NL); KRUEGER, Sascha, Eindhoven (NL)
(74) Representative: Philips Intellectual Property & Standards

(57) **Abstract**

The present invention relates to device, system and method of preparing a medical imaging examination of a patient. The method comprises obtaining patient-related information related to the medical imaging examination; adapting one or more parameters of the medical imaging examination and/or a medical imaging apparatus used for the medical imaging examination based on the obtained patient-related information; and generating and providing guidance information based on the obtained patient-related information, the guidance information being configured to guide the patient how to prepare for the medical imaging examination.

## Description

### FIELD OF THE INVENTION

The present invention relates to a method, device and system of preparing a medical imaging examination of a patient.

### BACKGROUND OF THE INVENTION

One of the main challenges for medical imaging today is the lack of experienced technicians, in particular given the continuously increasing demand. A potential solution is patient self-preparation, i.e., workflow guidance solutions that enable a partial or complete examination setup by the patient itself.

### SUMMARY OF THE INVENTION

It is an object of the present invention to provide a method, device and system of preparing a medical imaging examination of a patient that enable and support at least partial self-preparation of the medical imaging examination by the patient.

In a first aspect of the present invention a method of preparing a medical imaging examination of a patient, in particular for enabling self-preparation of a patient.is presented, the method comprising:
- obtaining patient-related information related to the medical imaging examination;
- adapting one or more parameters of the medical imaging examination and/or a medical imaging apparatus used for the medical imaging examination based on the obtained patient-related information; and
- generating and providing guidance information based on the obtained patient-related information, the guidance information being configured to guide the patient how to prepare for the medical imaging examination.

In a further aspect of the present invention a corresponding device is presented, the device comprising a processing unit configured to:
- obtain patient-related information related to the medical imaging examination;
- adapt one or more parameters of the medical imaging examination and/or a medical imaging apparatus used for the medical imaging examination based on the obtained patient-related information; and
- generate and provide guidance information based on the obtained patient-related information, the guidance information being configured to guide the patient how to prepare for the medical imaging examination.

In a still further aspect of the present invention a corresponding system is presented, the system comprising:
- an acquisition unit configured to acquire patient-related information related to the medical imaging examination;
- a device as disclosed herein for preparing a medical imaging examination of a patient and providing guidance information configured to guide the patient how to prepare for the medical imaging examination; and
- an output unit configured to output the guidance information to the patient.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, computer program and medium have similar and/or identical preferred embodiments as the claimed method, in particular as defined in the dependent claims and as disclosed herein.

The present invention is based on the recognition that a substantial obstacle for patient self-preparation is correct placement of components of the medical imaging apparatus, such as magnetic resonance (MR) coil needed for a specific MR imaging (MRI) examination. Especially for components placed below the patient's body, such as specific MR body coils, correction of their position can be challenging once the patient is already lying on the patient support. On the other hand, visual estimation of the suitable component position on an empty patient support is a difficult task even for experienced technicians. In addition, for components placed above the patient's body, the component must be reachable for the patient while already lying on the patient support, which is currently difficult in a typical medical imaging examination environment, such as a typical MRI scanner room. Furthermore, the patient is often not able to take the right position for the imaging examination but is typically guided by experienced staff in the examination room. Still further, the examination apparatus, e.g. an MRI scanner, and the examination operation, e.g. an imaging protocol, are typically adapted to the patient and the desired examination by an experienced technician.

The present invention makes use of patient-related information to provide guidance to the patient for self-preparation and for automated adaptation of medical imaging examination and/or the medical imaging apparatus. Hereby, the self-preparation and the automated adaptation may be supported fully or at least partly, i.e., some of the required steps may still be performed manually by support staff. In other embodiments, however, a fully automated process may be envisaged, e.g., as a self-imaging procedure for standard imaging examination where the patient performs a complete self-preparation and where the imaging examination is prepared and performed in a fully automated manner.

According to a preferred embodiment, the patient-related information is obtained from one or more of:
- one or more cameras monitoring one or more of an examination room, a preparation room, a waiting room, and an entrance;
- a patient database holding patient-related information;
- an input unit configured to input patient-related information; and.
- a measurement unit configured to acquire patient-related information.

Cameras are often provided anyhow to monitor certain areas. From camera images different pieces of patient-related information can be easily obtained using conventional image processing, such as body height, body shape, gender, body proportions, etc. Furthermore, even the patient may be identified, and patient-related information may then be obtained from a database if the patient has undergone a medical imaging examination before or if patient-related information is stored in a database, such as a database of medical records of a patient of a hospital or doctor's office, a database of electronic health records of patients, e.g. of health insurance companies, etc. Patient-related information may also be inputted by the patient, e.g. via an input terminal, a keypad, an interactive voice controlled terminal, etc. Different measurement units may be used to acquired patient-related information, such as a scale to obtain the patient's weight, a height measurement unit (e.g. via an optical or mechanical measurement element operated by the patient), etc.

The patient-related information may include different pieces of information useful for the desired adjustment of parameter and/or support for self-preparation. These pieces of information may include one or more of size, weight, age, age group, body shape, gender, mobility restrictions, anatomical constraints, presence of implants or other potentially metallic items, image data of the patient, anatomical keypoints and/or keypoint positions of the patient, medications, health status, medical history, and intolerances. These pieces of patient-related information may be obtained from different sources as explained above.

In an embodiment one or more parameters of the medical imaging apparatus may be adjusted based on the patient-related information, which parameters may include one or more software-related parameters (e.g. the type of imaging protocol, the anatomical parts to be imaged, length, intensity, etc.) and/or one or more hardware-related parameters (e.g. position of the patient support, position and kind of auxiliary components (such as MRI coils, x-ray lead protection, etc.).

In an embodiment that can be implemented by use of conventional image processing algorithms, the method further comprises:
- determining keypoint positions of the patient based on the patient-related information, in particular based on image data of the patient;
- determining patient height based on the determined keypoint positions;
- determining a desired patient pose for the medical imaging examination based on the determined keypoint positions and/or the determined patient height; and
- using the determined desired patient pose for adapting one or more parameters of the medical imaging examination and/or the medical imaging apparatus and/or for generating and providing the guidance information.

The keypoint positions may be characteristic anatomical positions of a person, such as head, face, shoulders, arms, hands, legs, torso, joints, ankles, etc. Typically, such characteristic anatomical positions can be detected in camera images of a person using recognition software or using a software for generating a skeleton model of a person. From those keypoint positions and knowledge about the intended imaging examination and the available imaging apparatus, the pose that the patient should adopt during the examination can be computed and provided as guidance information to the patient. Alternatively or in addition, parameters of the intended imaging examination and the available imaging apparatus may be adjusted to make sure that the patient is able to adopt the desired patient pose during the examination.

Hereby, in addition, the method may comprise generating and providing, based on the determined desired patient pose, guidance information being configured to indicate the patient pose that the patient should adopt during the medical imaging examination and/or to indicate the position and/or orientation how the patient should place one or more components of the medical imaging apparatus. This further supports the patient to perform self-preparation for the examination. The guidance information may, e.g., be provided as visual information on a display or through illumination of guidance elements or parts of the examination apparatus. For instance, it may be shown on a display how and where the patient should lay down on the patient support and/or how and where MR body coils should be placed on the patient support or on the patient's body.

Furthermore, the method may further comprise adapting, based on the determined desired patient pose, one or more of:
- height and/or position of a patient support,
- height and/or position of an auxiliary table supporting one or more components of the medical imaging apparatus to be used for the medical imaging examination,
- position and/or movement of an auxiliary means such as a robotic arm,
- a visual indication of a cabinet holding one or more components of the medical imaging apparatus to be used for the medical imaging examination, and
- one or more imaging parameters.

The position and kind of components that may be provided may include accessories like blankets, injectors, MR coils, x-ray lead protection, alarm buttons, remote control, etc. In general, based on the determined patient pose, any element of the examination process and/or the examination apparatus that may be needed may be automatically selected and/or provided and/or patient pose to ensure a large degree of self-preparation and automation.

In a preferred embodiment a trained algorithm or computing system that has been trained on a plurality of pieces of patient-related information, in particular on a plurality of images of the patient, may be used to determine the keypoint positions of the patient. Such a trained algorithm or computing system may be a conventionally programmed algorithm or computing system, but may alternatively be a learning system, a neural network, a convolutional neural network or a U-net-like network. In an exemplary implementation, a large number of pieces of patient-related information, for instance a large number of images of a large number of persons, preferably with annotated keypoint positions, may be used as training data, for which the detection of keypoint positions may be trained using standard techniques such as stochastic gradient descent.

In another embodiment, the method may further comprise:
- generating a patient model, in particular a 2D or 3D patient model, of the patient based on the patient-related information;
- combining the generated patient model with an apparatus model, in particular a 2D or 3D apparatus model, of the medical imaging apparatus or of one or more components of the medical imaging apparatus; and
- adapting one or more parameters of the medical imaging examination and/or the medical imaging apparatus and/or generating and providing the guidance information based on the combination of the generated patient model and the apparatus model.

This may further improve the accuracy and of the adaptation and of the guidance information and contributes to achieve the desired self-preparation.

The device according to the present invention comprises a processing unit that is configured to carry out the steps of the method as disclosed herein. The device may be a standalone device, such as a computer, laptop, tablet, smartphone, workstation, but may also be part of the image examination apparatus or a control station that controls the imaging examination. Even further, the device may be part of a cloud or a server, which - based on the obtained patient-related information - computes the desired guidance information and adaptations of the examination and apparatus, which are returned to the patient and the apparatus, respectively. The processing unit may be a single programmed processor.

The system according to the present invention comprises an acquisition unit, a device as disclosed herein and an output unit. The system may be implemented as a single device, e.g. a computer or workstation within the examination room, but may also be implemented as two or more separate devices.

In preferred embodiments, the acquisition unit may include one or more of a camera, a database connection, and an input unit, and the output unit may include one or more of a display, a projector, and a loudspeaker.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system and a device according to the present invention.
Fig. 2 shows a flowchart of a first embodiment of a method according to the present invention.
Fig. 3 shows a flowchart of a second embodiment of a method according to the present invention.
Fig. 4 shows an exemplary image of patient.
Fig. 5 shows an exemplary image of a 3D model of a patient and of a patient support.

### DETAILED DESCRIPTION OF EMBODIMENTS

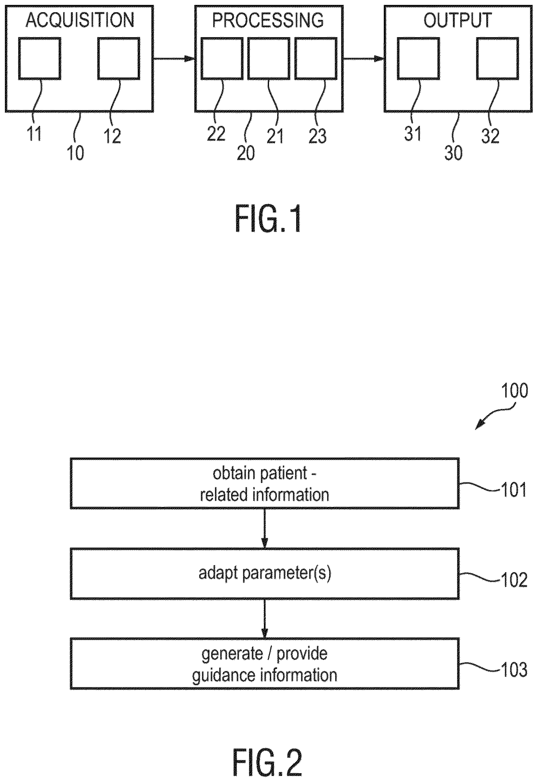

Fig. 1 shows a schematic diagram of an embodiment of a system 1 and a device 20 for preparing a medical imaging examination of a patient according to the present invention. The system 1 and device 20 are particularly designed according to the present invention to enable a patient to - at least partly - self-prepare for the upcoming medical imaging examination, e.g. an MRI examination or an X-ray or CT examination, and to - at least partly - adapt the medical imaging examination and/or the medical imaging apparatus to the patient and the desired kind of imaging examination, e.g. to the body part that shall be examined, the type of examination, etc. In this way, efforts of technical staff that typically carries out all steps of preparing the patient and adapting the medical imaging examination and/or the medical imaging apparatus can be reduced up to a minimum of steps that have to be carried out by experts and cannot be carried out by patients or to certain checks if the patient has self-prepared correctly and the adaptation of any parameters is correct. In certain application it may even be envisaged that a medical imaging examination may be carried out in a fully automated manner and the patient self-prepared completely.

The system 1 comprises an acquisition unit 10 for acquiring patient-related information related to the medical imaging examination. The acquired patient-related information is provided, e.g. through wireless or wired communication, to the device 20
for preparing a medical imaging examination of the patient and for providing guidance information configured to guide the patient how to prepare for the medical imaging examination. The guidance information is provided to an output unit 30 for outputting it to the patient for guiding the patient how to self-prepare for the desired medical imaging examination.

The acquisition unit 10 may be a single device or may comprise two or more device of the same type or of different types, i.e., there may be two or more acquisition sub-units 11, 12 in practical scenarios. In embodiments the acquisition unit 10 may comprises one or more cameras that are monitoring one or more of an examination room, a preparation room, a waiting room, and an entrance. For instance, two wall-mounted cameras (RGB, IR, ...) may be used that provide a continuous data stream of the examination room's door. Further options for the acquisition unit 10 include a patient database holding patient-related information (e.g. a hospital's EHR (electronic health record) database, a database of patient records stored on a secure server, etc.), an input unit for inputting patient-related information (e.g. a keypad, a touchscreen, etc.) and a measurement unit to acquire patient-related information (e.g. a scale to obtain the patient weight, a height measurement device, etc.).

The patient-related information may include one or more different pieces of information that may be relevant for the medical imaging examination and may thus be useful for preparing the guidance information and/or for adapting one or more parameters of the medical imaging examination and/or a medical imaging apparatus. One or more of the following pieces of patient-related information of the patient may thus be acquired by the acquisition unit: size, weight, age, age group, body shape, gender, mobility restrictions, anatomical constraints, presence of implants or other potentially metallic items, image data of the patient, anatomical keypoints and/or keypoint positions of the patient, medications, health status, medical history, and intolerances.

Patient-related information may be acquired in advance, e.g. online via a query or list of questions to be answered by the patient at home or at a waiting room, and/or may be acquired when entering the examination room. Depending on the desired medical imaging examination, it may be selected which pieces of patient-related information shall be acquired. For instance, some pieces of patient-related information may be mandatory and other pieces of patient-related information may be optional.

The output unit 30 may include one or more of a display, a projector, and a loudspeaker, i.e. there may a single output unit 30 or two or output sub-units 31, 32. For instance, a monitor may be provided in the examination room that shows images and/or text to the patient and/or provides spoken instructions guiding the patient what to do to prepare himself or herself and/or how to prepare the examination apparatus or any components thereof for the intended examination. For instance, an image may be displayed showing how the patient shall lay down on the patient support (e.g. supine or prone position, legs straight or bent, etc.) and/or if and which additional components shall be used and how it shall be arranged (e.g. how an MR body coil shall be arranged under the torso or around the knee, where an x-ray lead protection shall be arranged, if hearing protection shall be worn, etc.).

The device 20 includes a processing unit 21, which may be implemented by any kind of processing element, such as a processor, computer, tablet, smartphone, workstation, etc., in particular in hard- and/or software. In an embodiment it may be implemented as a programmed processor, e.g. a processor running an algorithm or software. In an embodiment, a trained computer system or algorithm (e.g. a learning system, a neural network, a convolutional neural network or a U-net-like network) may be used for implementing the method carried out by the device or at least parts of the method.

The device 20 gets as input the acquired patient-related information and may thus include one or more input interfaces 22 that are configured to obtain (i.e. receive or retrieve) the various pieces of patient-related information from the acquisition unit 10 or the respective acquisition sub-unit(s) 11, 12. The device 20 may further include one or more output interfaces 23 that are configured to provide as output (i.e. transmit or make available for retrieval) the guidance information and any adaptations of the parameters of the medical imaging examination and/or a medical imaging apparatus, which may be provided to a control unit (e.g. a workstation or computer) of the medical imaging apparatus that controls the medical imaging apparatus to carry out the desired examination. The input interface(s) 22 and the output interface(s) 23 may be configured to obtain / provide the respective data and information in a wireless or wired way, e.g. via cable, Wi-Fi, Bluetooth, or any communications or IP network.

The device 20 is configured to carry out a method 100 of preparing a medical imaging examination of a patient according to the present invention as illustrated in the flowchart shown in Fig. 2. In a first step 101, patient-related information related to the medical imaging examination is obtained. In a second step 102, one or more parameters of the medical imaging examination and/or a medical imaging apparatus used for the medical imaging examination are adapted based on the obtained patient-related information. In a third step 103, which may be carried out before, simultaneously with or after step 102, guidance information is generated and provided based on the obtained patient-related information, the guidance information being configured to guide the patient how to prepare for the medical imaging examination.

Parameters of the medical imaging examination and/or the medical imaging apparatus may include one or more software-related parameters and/or one or more hardware-related parameters. These parameter may, e.g., include the patient support height, coil position on the patient support, position of a movable auxiliary table (which moves autonomously or is moved manually by the patient) that support components to be used by the patient for the examination (e.g. MR coils, x-ray lead protection, hearing protection, alarm button, knee pad, etc.), radiation dose, radiation direction, examination area, type of MR sequence, etc.

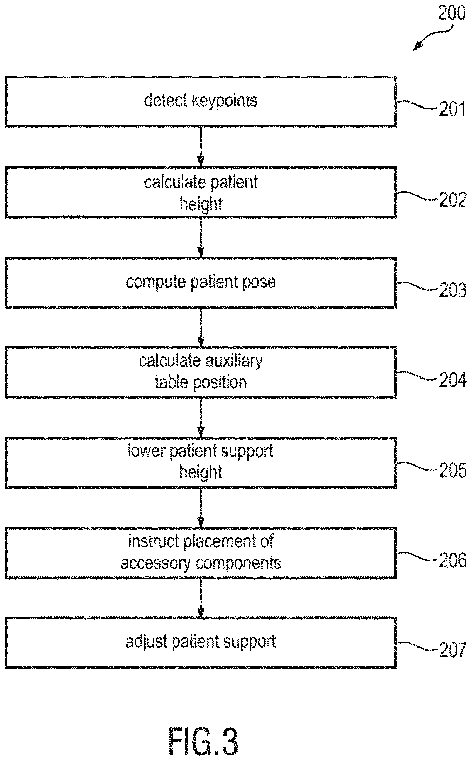

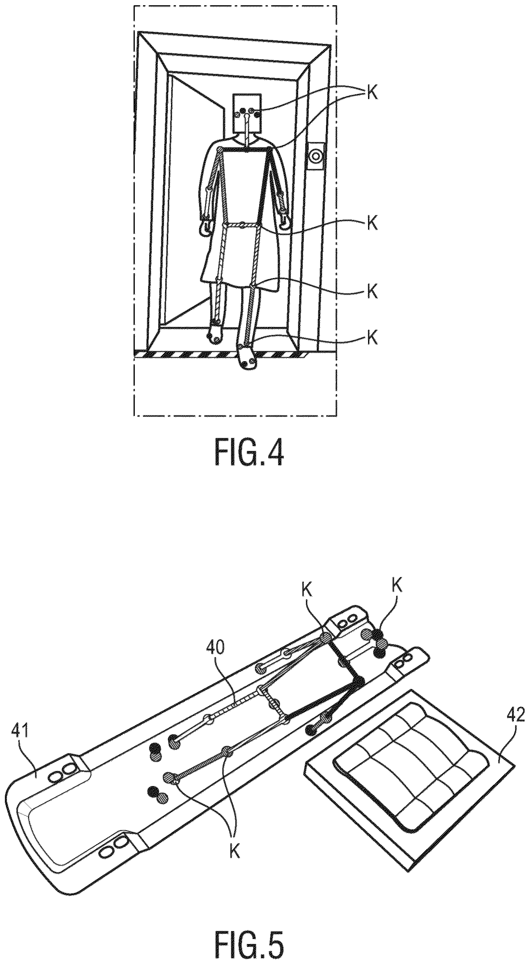

Fig. 3 shows a flowchart of a second embodiment of a method 200 according to the present invention. In a first step, 201, once a patient enters the examination room, e.g. a room of an MRI or CT scanner, a dedicated neural network detects pre-defined body keypoints on camera images of the patient. Identification of the patient may be realized by detecting a specific piece of clothing that is exclusively used for patients at the given site, e.g., a particular patient gown, or by face detection or detection of an ID tag or other tag word by the patient. This may be realized by standard pattern detection algorithms or an additional dedicated neural network for object detection. Since the camera positions and orientations are known, triangulation can be used to infer 3D keypoint positions. Keypoints may e.g. be characteristic anatomical points like face, eyes, ears, arms, shoulders, joints, ankles, legs, knees, etc. The dedicated neural network may be trained by a large number of camera images of different person to learn how to detect keypoint positions of different types of persons. Fig. 4 shows an exemplary image of a patient entering the examination room with detected body keypoints K, some of which have been indicated.

In a second step 202, based on the detected 3D keypoint positions, the patient height is calculated, e.g. using a simple linear model. This information is then used to move the patient support to a height that facilitates easy placement of the patient and any accessory components, such as an MR body coil in case of an MRI examination.

In a third step 203, using body pose transformations that obey pre-defined anatomical constraints (e.g., maximum joint rotation angles), the detected 3D keypoint positions are converted to the desired patient pose required for the desired type of examination (e.g., feet first / supine position). For MR coils with a part that is placed below the patient's body (e.g., current knee coils), these converted 3D keypoint positions are used to calculate the suitable coil position on the patient support. This position is shown to the patient to guide coil placement. The patient may further be instructed to plug the coil cable into the suitable socket (if needed).

In a fourth step 204, for other accessory components, like other MR coil parts in case of an MRI examination or x-ray lead protection in case of an x-ray or CT examination, that are placed on top of the patient's body (e.g., top part of knee coil, anterior coil), the converted 3D keypoint positions are used to calculate the suitable position of an auxiliary table next to the patient support. The patient may then be instructed to place the accessory component on the auxiliary table, move it to the calculated position (or the table is motorized and moves autonomously to the calculated position without patient interaction) and plug the coil cable into the suitable socket (if needed).

Fig. 5 shows an exemplary image of a patient model 40 lying on a 3D model the patient support 41, which shows the desired patient pose (head first, supine in this case) computed from the keypoints. The patient model 40 including the keypoints K is placed onto a 3D model of the patient support 41. Based on the keypoint positions K on the patient support 41, the position of the auxiliary table 42 for self-placement of accessory components is calculated.

In a fifth step 205, using knowledge of the patient's height, the patient support height is lowered to a suitable level. The patient is instructed to get onto the patient support. Using visual feedback, the patient is guided to assume the body pose required for the prescribed type of examination.

In an optional sixth step 206, the patient is instructed to place the necessary accessory component, e.g. an MR body coil, onto his/her body. Visual feedback is provided on the screen to guide this placement.

In a seventh step 207, the patient support is automatically moved to the suitable position to start the examination (auto-isocentering).

Optionally, before the examination is started, further camera images may be acquired during the self-preparation, to check if the patient carried out the instructed steps correctly or if any adjustments still need to be made. Some feedback may be provided to the patient that the self-preparation is correct or that the patient has to make some corrections, e.g. to lay down differently on the patient support or to place an accessory component at a different place or with a different orientation.

In another optional step in this embodiment the determined desired patient pose may be used for adapting one or more parameters of the medical imaging examination and/or the medical imaging apparatus. For instance, imaging parameter of the medical imaging examination may be set accordingly, the imaging sequence may be chosen, radiation dose and direction may be chosen, etc.

Further embodiments and modifications to the embodiments described above can be envisaged. For instance, a projector may be used to highlight the correct location of any accessory component on the patient support. A single RGB camera may be used in combination with a dedicated neural network that provides 3D keypoint positions from a single 2D image. Guidance for placement of cushions on the patient support may also be shown to the patient.

As an alternative to the described patient identification system based on clothing items, a set of images of the operator staff can be stored in a database. Any person entering the examination room is then compared to this database using standard person identification algorithms. If no database match is found, the person is identified as a patient.

The cabinet with the correct accessory component for the prescribed examination may be highlighted using a dedicated lighting solution to support selection and placement of the accessory component.

Some accessory components, e.g. large MR coils, are relatively heavy, which may complicate lifting from the auxiliary table and placement onto the body by the patient itself. To facilitate placement, a robotic arm may be attached to the auxiliary table to support the patient during this process by reducing the effective weight of the accessory component.

The method, device and system according to the present invention enable patient self-preparation for a medical imaging examination at least to a certain extent or even fully. This reduces the required efforts of technical experts that usually prepare the patient, the examination apparatus and the examination process. The present invention may be applied in various imaging modalities, like MR (including PET-MR and MR-Linac), CT, X-ray, or any other imaging modalities. It may even be envisaged to use the present invention for self-preparation of the patient for radiation therapy examinations. Placement guidance of components may be used for various components, such as MR coils, x-ray lead protection, patient support, auxiliary table, etc. Various pieces of patient-related information may be used for computation of the adaptation and guidance, such as patient height and prediction of 3D body keypoint locations on the patient support.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

It is to be understood that any of the previous steps described in relation to embodiments and/or training steps described above can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. In embodiments, a computer system performs logical processing based on digital signals received via an analogue-to-digital converter.

Some portions of the description are presented in terms of symbolic representations of operations on non-transient signals stored within a computer memory. These descriptions and representations are used by those skilled in the data processing arts to convey the substance of their work most effectively to others skilled in the art. Such operations typically require physical manipulations of physical quantities. Usually, though not necessarily, these quantities take the form of electrical, magnetic, or optical signals capable of being stored, transferred, combined, compared, and otherwise manipulated. It is convenient at times, principally for reasons of common usage, to refer to these signals as bits, values, elements, symbols, characters, terms, numbers, or the like. Furthermore, it is also convenient at times to refer to certain arrangements of steps requiring physical manipulation of physical quantities as modules or code devices, without loss of generality.

The computer system further includes a main memory and a static memory, where memories in the computer system communicate with each other and the processor via a bus. Either or both main memory and the static memory may be considered representative examples of the memory of the controller, and store instructions used to implement some, or all aspects of methods and processes described herein. Memories described herein are tangible storage mediums for storing data and executable software instructions and are non-transitory during the time software instructions are stored therein. The main memory and the static memory are articles of manufacture and/or machine components. The main memory and the static memory are computer-readable mediums from which data and executable software instructions can be read by a computer (or e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM (Compact Disk - Read Only Memory)), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices. The memory may store various software applications including computer executable instructions, that when run on the processor, implement the methods and systems set out herein. Other forms of memory, such as a storage device and a mass storage device, may also be included and accessible by the processor (or processors) via the bus. The storage device and mass storage device can each contain any or all of the methods and systems discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

## Claims

1. Method of preparing a medical imaging examination of a patient, the method comprising:
- obtaining patient-related information related to the medical imaging examination;
- adapting one or more parameters of the medical imaging examination and/or a medical imaging apparatus used for the medical imaging examination based on the obtained patient-related information; and
- generating and providing guidance information based on the obtained patient-related information, the guidance information being configured to guide the patient how to prepare for the medical imaging examination.

2. Method according to claim 1,
wherein the patient-related information is obtained from one or more of:
- one or more cameras monitoring one or more of an examination room, a preparation room, a waiting room, and an entrance;
- a patient database holding patient-related information;
- an input unit configured to input patient-related information; and.
- a measurement unit configured to acquire patient-related information.

3. Method according to any one of the preceding claims,
wherein the patient-related information includes one or more of size, weight, age, age group, body shape, gender, mobility restrictions, anatomical constraints, presence of implants or other potentially metallic items, image data of the patient, anatomical keypoints and/or keypoint positions of the patient, medications, health status, medical history, and intolerances.

4. Method according to any one of the preceding claims,
wherein the one or more parameters of the medical imaging apparatus include one or more software-related parameters and/or one or more hardware-related parameters.

5. Method according to any one of the preceding claims, further comprising:
- determining keypoint positions of the patient based on the patient-related information, in particular based on image data of the patient;
- determining patient height based on the determined keypoint positions;
- determining a desired patient pose for the medical imaging examination based on the determined keypoint positions and/or the determined patient height; and
- using the determined desired patient pose for adapting one or more parameters of the medical imaging examination and/or the medical imaging apparatus and/or for generating and providing the guidance information.

6. Method according to claim 5, further comprising:
- generating and providing, based on the determined desired patient pose, guidance information being configured to indicate the patient pose that the patient should adopt during the medical imaging examination and/or to indicate the position and/or orientation how the patient should place one or more components of the medical imaging apparatus.

7. Method according to claim 5 or 6,
further comprising adapting, based on the determined desired patient pose, one or more of:
- height and/or position of a patient support,
- height and/or position of an auxiliary table supporting one or more components of the medical imaging apparatus to be used for the medical imaging examination,
- position and/or movement of an auxiliary means such as a robotic arm,
- a visual indication of a cabinet holding one or more components of the medical imaging apparatus to be used for the medical imaging examination, and
- one or more imaging parameters.

8. Method according to any one of claims 5 to 7,
wherein a trained algorithm or computing system that has been trained on a plurality of pieces of patient-related information, in particular on a plurality of images of the patient, is used to determine the keypoint positions of the patient.

9. Method according to claim 8,
wherein a learning system, a neural network, a convolutional neural network or a U-net-like network is used as trained algorithm or computing system.

10. Method according to any one of claims 5 to 9, further comprising:
- generating a patient model, in particular a 2D or 3D patient model, of the patient based on the patient-related information;
- combining the generated patient model with an apparatus model, in particular a 2D or 3D apparatus model, of the medical imaging apparatus or of one or more components of the medical imaging apparatus; and
- adapting one or more parameters of the medical imaging examination and/or the medical imaging apparatus and/or generating and providing the guidance information based on the combination of the generated patient model and the apparatus model.

11. Device (20) for preparing a medical imaging examination of a patient, the device comprising a processing unit (21) configured to:
- obtain patient-related information related to the medical imaging examination;
- adapt one or more parameters of the medical imaging examination and/or a medical imaging apparatus used for the medical imaging examination based on the obtained patient-related information; and
- generate and provide guidance information based on the obtained patient-related information, the guidance information being configured to guide the patient how to prepare for the medical imaging examination.

12. System (1) for preparing a medical imaging examination of a patient, the system comprising:
- an acquisition unit (10) configured to acquire patient-related information related to the medical imaging examination;
- a device (20) according to claim 11 for preparing a medical imaging examination of a patient and providing guidance information configured to guide the patient how to prepare for the medical imaging examination; and
- an output unit (30) configured to output the guidance information to the patient.

13. System according to claim 12,
wherein the acquisition unit (10) includes one or more of a camera, a database connection, a measurement unit and an input unit.

14. System according to claim 12 or 13,
wherein the output unit (30) includes one or more of a display, a projector, and a loudspeaker.

15. Computer program comprising program code means for causing a computer to carry out the steps of the method as claimed in any one of claims 1 to 10 when said computer program is carried out on the computer.
